# EUROPEAN PATENT SPECIFICATION

(11) **EP 4 479 486 B1**
(45) Date of publication and mention of the grant of the patent: **06.05.2026**
(21) Application number: 23710929.3
(22) Date of filing: 17.02.2023
(51) Int. Cl.: C09K 11/06, G21K 4/00, G01T 1/203

(54) **ORGANIC SCINTILLATOR**
ORGANISCHER SZINTILLATOR
SCINTILLATEUR ORGANIQUE

(30) Priority: 17.02.2022 IT 202200002996
(43) Date of publication of application: 25.12.2024
(73) Proprietor: Università degli Studi di Roma "La Sapienza", 00185 Roma (IT); Museo Storico della Fisica e Centro Studi e Ricerche "Enrico Fermi", 00184 Roma (RM) (IT)
(72) Inventor: MATTIELLO, Leonardo, 00185 Roma (RM) (IT); PATERA, Vincenzo, 00185 Roma (RM) (IT); BELARDINI, Alessandro, 00185 Roma (RM) (IT); ROCCO, Daniele, 00185 Roma (RM) (IT); MARAFINI, Michela, 00184 Roma (RM) (IT)
(74) Representative: Primiceri, Maria Vittoria
(86) International application number: PCT/IB2023/051452
(87) International publication number: WO 2023/156957

(56) References cited:
- US-A1- 2021 317 364

## Description

### Technical field

The present invention relates to organic scintillators, more particularly to solid and liquid organic scintillators.

The invention also relates to chemical compounds which can be used in organic scintillators.

### Background

Scintillators are mainly used in physics as particle detectors, mainly to detect charged and neutral particles. They are used for the simple counting of particles or, for instance, for measuring the time of flight, from which it is possible to obtain the speed of the particle and its mass. Scintillators can also be used in neutron physics, X-ray protection, nuclear monitoring, and gas detection. Other applications of scintillators include CT (Computed Tomography) scanners and gamma ray cameras used in medical imaging. The use of a scintillator in combination with a photomultiplier tube is widely used in handheld remote measurement devices used for the detection and measurement of radioactivity and for monitoring nuclear material. Scintillation detectors are used in the petroleum industry as detectors for gamma rays.

A particle passing through the scintillator loses energy by transferring it to the latter with physical mechanisms which is then followed by the emission of photons. In amorphous (plastic, liquid) scintillators, the energy transferred is used to excite the molecules it is made of, which, when de-excited, emit photons with an exponential time pattern. In the most common scintillators, the emission occurs mainly in the violet, with times ranging from nanoseconds to microseconds. These photons are then transmitted, through a suitable light guide, to the photocathode of the photomultiplier. Here the photons release, by photoelectric effect, electrons, which are then accelerated and focused on the first dynode. The ratio between the number of photoelectrons produced and the number of photons incident on the photocathode is called "quantum efficiency of the photocathode". For every primary photoelectron that collides with a dynode, 2 to 5 secondary photoelectrons can be emitted. By introducing, for instance, 14 multiplication stages, multiplication factors of about one billion are reached. The collected charge (pulse integral) and the pulse amplitude are proportional to the energy deposited in the scintillator. Scintillators can be organic or inorganic.

A scintillator is a material which exhibits the phenomenon of scintillation (the property of luminescence) when excited by ionizing radiation. Luminescent materials, when struck by a particle, absorb its energy and sparkle (that is, they release the absorbed energy in the form of light).

A scintillation detector or scintillation counter is obtained when a scintillator is coupled to an electronic light sensor such as a photomultiplier tube (PMT), photodiode or silicon photomultiplier (SiPM). PMTs absorb the light emitted by the scintillator and emit it in the form of electrons through the photoelectric effect. Subsequent multiplication of those electrons (sometimes called photoelectrons) produces an electrical impulse that can then be analyzed and provide meaningful information about the particle that originally hit the scintillator.

A plastic scintillator is formed by a solution of organic scintillating material dissolved in a solvent which is subsequently polymerised, thus becoming a solid solution. Very often a secondary solute is also added due to its "shifting" properties of the wavelength of the light produced.

Plastic scintillators offer a very fast signal with a decay constant of about 2-3 ns and a light output proportional to the energy release. One of the major advantages of plastic scintillators is their flexibility, which makes them easy to manipulate; their not excessive cost makes them particularly useful if large volumes of scintillators are required.

Plastic scintillators used in high energy physics are solutions of fluorophores in a plastic matrix based on aromatic compounds. Virtually all plastic scintillators contain polyvinyltoluene, polystyrene, or acrylic polymers as a base. Acrylic is non-aromatic and therefore has a very low scintillation efficiency. It becomes acceptable when naphthalene is dissolved in percentages of the order of 5-20%.

The plastic matrix represents the component sensitive to ionization (i.e. the "scintillator"). In the absence of a shifter, the base would emit UV light with a low attenuation length (a few mm). To obtain longer attenuation lengths a fluorophore in high concentrations (1 wt% or more) is dissolved in the plastic matrix. https://physicsopenlab.org/2017/08/10/cristalli-scintillatori/.

The operation principle of plastic scintillators consists in the absorption of the energy of the incident radiation by an inexpensive polymer matrix [for instance, poly(vinyltoluene) (PVT)] followed by a subsequent rapid transfer of this energy to a fluorescent primary dopant. Sometimes, to minimize self-absorption phenomena of the primary dopant, a secondary dopant (wavelength shifter) is added with the aim of obtaining the wavelength shift of the scintillation system towards higher values. The emitted photons are collected by a photodetector such as a photomultiplier tube (PMT) or photodiode from which a pulse is generated that is proportional to the type and energy of the incident radiation.

In recent years, research groups have begun to study and describe methods to improve the sensitivity of plastic scintillators to distinguish fast neutron signals from background radiation. A simple and reproducible method to achieve this effect has emerged, and it relies on the introduction of an increased amount of highly soluble fluorescent primary dopant (over-doping) into a standard formulation of plastic scintillator to induce interactions that allow for discrimination analysis of the pulse shape (PSD).

For instance, US patent document 2021/0317364A1 describes plastic scintillators containing, in addition to a lithium-based compound, also a primary dye or dopant and, to a lesser extent, a secondary dye or wave shifter, in order to maximize the difference in optical response between thermal neutrons, fast neutrons and photons. However, the choice of primary dyes or dopants, combined with the secondary dyes or wave shifters, leads to a non-optimal response of the scintillator since the incident radiation, interacting more with these primary dopants, provides either a slow signal or a signal with a double component of which the slower one deteriorates the fast one, thus leading to an unsatisfactory temporal resolution of the scintillator. In fact, the scintillators described in US 2021/0317364A1 are used in the techniques which allow to induce interactions which allow the analysis of the discrimination of the pulse shape (PSD) but the chosen components (primary dopant and shifter) heavily slow down the response of the scintillator, as well as having negative effects on its transparency. In fact, the compositions of the transparent materials illustrated in the figures are never exemplified, on the other hand the compositions indicated in fig. 9, in particular the one with 30% by weight of PPO is certainly opaque as, according to the inventors, a ter-phenyl-based scintillator would be opaque.

In general, a scintillator is a material capable of absorbing the kinetic energy of a radiation and converting a part of it into optical photons. In organic plastic scintillators this task falls to the primary dopants, organic molecules which however must possess specific requisites. They must show high solubility in organic solvent precursors of plastic materials, such as for instance 4-vinyltoluene or styrene. In fact, the possibility of reaching high concentrations of primary dopant allows to significantly improve the performance of these materials in terms of Light Output (LO). Furthermore, since the photons generated by the scintillation phenomenon must escape from the plastic material to be collected by a suitable detector device (photo-detector) and to be analysed, it is essential to obtain highly homogeneous and transparent plastic objects, even when the concentration of the primary dopant is very high (>3% by weight as compared to the polymeric matrix).

In particular, the molecules used as dopants must show very intense fluorescence phenomena demonstrating that they do not suffer from problems of "quenching" of the luminescence phenomenon due to self-absorption, which emerges more pronounced as the doper concentration increases.

Furthermore, the emission band of the primary dopant must be located outside the absorption region of the polymeric matrix. The most common aromatic matrices, such as PVT (polyvinyltoluene) or PS (polystyrene), absorb in a region of the electromagnetic spectrum starting at approximately 380 nm. In some cases the primary dopant can show a significant loss of light transmission in the spectrum region wherein it emits, drastically decreasing the effective Light Output. In these cases it is possible to proceed with the addition of a secondary dopant (wavelength shifter) which has the role of moving the emitted scintillation in a region where there are no self-absorption phenomena. However, it should be remembered that the most commonly used secondary dopants such as POPOP (1,4-Bis(5-phenyl-2-oxazolyl)benzene) and 9,10-diphenylanthracene have low solubility in 4-vinyltoluene and styrene, which could potentially negatively affect the transparency and homogeneity of the final plastic object.

It is worth mentioning that the most commonly used scintillation light reading and gathering devices, such as photomultiplier tubes, traditionally operate in a detection window in the blue region of the optical spectrum, and it is therefore particularly favorable that the emission band of the primary dopant is between 300 and 450 nm.

When defining the requirements for the innovative development of a plastic scintillator, it is essential to clarify its main end use. In particular, the aim is to create detectors which provide a signal as quickly as possible following the passage of the radiation. The primary motivation for optimizing this feature is that in general, the faster the time response of the detector, the more accurately the arrival time of radiation at the photodetector can be determined. In this perspective, the crucial parameters are the scintillation time, the rise time, the decay time and the aforementioned light output.

It is important to underline the importance of the transparency and hardness characteristics of the final scintillator material for its effective application as a detector of ionizing radiation. The plastic scintillator sample must have hardness characteristics such as to allow its use in particle detection devices, so that its geometric shape must be guaranteed and constant over time under operating conditions. In addition, complete polymerization of the matrix will ensure no subsequent release of liquid material. At the same time, the transparency of the material ensures the efficiency of transmission of the light produced by it, increasing the luminous response.

The characteristic on which the inventors have concentrated is the fast time response of the scintillator to optimize the development of detectors which allow a precise determination of the arrival time of the incident radiation on the photodetector. Therefore, it is on this aspect that comparisons should be made with other developed systems.

In the document A. Sellinger et al. Highly Soluble p-Terphenyl and Fluorene Derivatives as Efficient Dopants in Plastic Scintillators for Sensitive Nuclear Material Detection Chem. Eur. J. 2017, 23, 8921-8931, doi: 10.1002/chem.201700877 characterization measurements of the temporal response of the scintillators are not performed; therefore, it is not possible to infer the response characteristics of the described scintillators and it is not possible to make a direct comparison.

The commercial scintillator taken as a reference for the light response in Sellinger's article (BC-408, Saint Gobain), leads to think that the authors are not interested in optimizing the temporal characteristics but, mainly, in applications of Pulse Shape Discrimination (PSD), as the BC-408 presents itself as a detector with a rise time equal to 900 ps and a decay time equal to 2100 ps, while the plastic scintillator to which the scintillators of the present invention are intended to be compared and which was used as a commercial reference (EJ-232, Eljen Technology) for the applications of the invention has characteristics decidedly superior to those of Sellinger's commercial reference in terms of response speed, having a rise time equal to 350 ps and a decay time equal to 1600 ps.

The same comments can be made for the patent document US 2021/0317364A1 mentioned above, which too, in fact, is aimed at applications of Pulse Shape Discrimination (PSD), due to the high intrinsic decay time of the material of which the scintillators described therein are made of.

To date, the fast commercial plastic scintillators are those of the BC-422 family and those of the EJ-232 family, marketed respectively by Saint Gobain and Eljen Technology. The scintillation time of these currently available plastic scintillators is 350 ps of rise time, 1600 ps of decay time and a pulse width of 1300 ps, with a light yield (LY) quoted at about 55% of the anthracene (to date in the literature and in commercial data-sheets, the light yield measurements are conventionally all referred as compared to the luminous response of anthracene). These characteristics pose an intrinsic limit to the possibility of providing more performing time detectors and the aim of the invention is to solve the problems set out here by providing transparent scintillators with improved characteristics.

Furthermore, although Sellinger in the above article claims to obtain scintillators with a primary dopant in a very high quantity compared to the polymer, however, with dopants with a fluorenic structure he is unable to achieve adequate transparency. See, for instance, figs. 11 where only with a compound with a fluorenic structure, SF, transparency is reached with 25% by weight of doping agent; all other transparency tests stop at amounts of fluorene dopants of 20% by weight or less. In addition, he illustrates PPO-based compositions which are heavily opaque already at 25% by weight.

Unless specifically excluded in the detailed description that follows, what is described in this chapter is to be considered as an integral part of the detailed description of the invention.

### Summary of the Invention

The invention is defined by the appended claim.

It is an object of the present invention to provide a scintillator with improved characteristics in terms of transparency and workability of the final scintillator material for effective application as a detector of ionizing radiation. It has in fact been found that an elongation of the alkyl chains which characterize the fluorophore compounds used in the present invention increases not only the response characteristics of the scintillators, but also and above all their transparency.

A further object of the invention is to provide highly transparent scintillators even with dopant quantities greater than 25% by weight as compared to the polymeric matrix.

The plastic scintillators produced according to the invention have mechanical characteristics such as to allow excellent workability in terms of cutting and polishing.

The scintillators according to the invention comprise a transparent polymeric material comprising a primary dopant the amount of which has been varied in a range ranging from 1% to 30% by weight as compared to the polymeric matrix. Preferred is a matrix based on poly(vinyl-toluene) (PVT) or cross-linked polystyrene.

Another object is to provide a plastic scintillator which can be worked like a commercial scintillator which allows, if inserted in a detection system, to reach better (i.e. lower) time resolutions than those obtainable today with the fastest commercial plastic scintillator. For instance, in the specific measurement condition presented in this work (cosmic rays and reading with commercial photomultipliers PMT Hamamatsu, H10721-20), the inventors' scintillators allow to obtain time resolutions much lower than 350 ps, for instance 81 ps compared to 123 ps obtained with a commercial plastic scintillator such as EJ-232 and EJ-204. The pulse width is also very fast, less than 20 ns, with a light yield (LO) approximately 55% or higher as compared to anthracene.

This time resolution is the result of a combination of the aforementioned parameters rise time, decay time, scintillation time, light yield (LY), transparency, emission spectrum. Preferably the time resolution is less than 120 ps, preferably less than 100 ps.

In fact, it has been verified that the best performing scintillators are those with the lowest number of components; in fact, those based on, or essentially consisting only of, a transparent plastic support and a primary doping agent (in addition to a possible secondary doping agent and any other minor components such as not to affect transparency) will have better characteristics, for instance in terms of transparency and light production.

Another object is to create a scintillator which, under the same measurement conditions, has a rise time, a decay time and a pulse width lower than those of the best performing fast scintillator existing on the market, against a superior Light yield (LO).

The scintillator according to the invention comprises a polymeric material, for instance polyvinyltoluene (PVT) or polystyrene (PS), a matrix based on polyvinyltoluene being preferred, possibly a crosslinking agent such as divinylbenzene (DVB) or ethylene glycol dimethacrylate (EGDMA), preferably divinylbenzene can be used.

According to one aspect of the invention a method is provided for producing a scintillator according to the invention.

The method includes the combination of a first fluorene dopant in an amount sufficient to ensure transparency, a secondary dopant in an amount from about 0.01 to about 2% by weight, a crosslinking agent preferably in an amount of at least 0.5% by weight, and the remainder is at least one polymerizable monomer to form a blend. Unless otherwise specified, the percentages are to be understood as % by weight.

Another object is to create a device for detecting particles (electrons, photons, protons, and more generally also leptons and hadrons) which uses the scintillator of the invention.

Further objects and aspects of the invention will become apparent from the following detailed description.

The claims describe preferred embodiments of the invention, forming an integral part of the present description.

### Brief description of the Figures

Further objects and advantages of the present invention will become clear from the detailed description that follows of an embodiment of the same (and of its variants) and from the annexed drawings given for purely explanatory and non-limiting purposes, wherein:
Figure 1 : illustrates artifacts in the form of tablets containing the primary dopant of 2N at 14% (left) and 7% (right) by weight (diameter 15 mm, height 10 mm).
Figure 2: shows a plastic scintillator in its final form containing the 2N primary dopant at 14% by weight. Left: Sample worked into the shape necessary for the measurements and inserted in the mechanical support which allows it to be coupled with the reading system (measurements 5 x 10 x 20 mm). Center: sample in transparency. Right: sample illuminated with a 365 nm UV lamp;
Figure 3: shows the artifacts in different percentages of primary dopant by weight, from top to bottom:
   2N, left to right at 7%, 14% and 30% respectively.
   1T at 7% left and 14% right, respectively
   1N respectively at 7% on the left, at 14% in the centre; the same sample at 14% is shown on the right, seen from the front;
   2B at 7% on the left and 14% on the right, respectively.
Figure 4: scheme of the experimental setup. Samples at various concentrations, from 7 to 30%, are placed one on top of the other and crossed by particles at minimum ionization which release the same amount of energy in each of them. With this experimental setup a measurement has been guaranteed which puts all the samples under the same irradiation conditions and allows a complete characterisation. Further details on the experimental measurements carried out and on the results obtained are reported below.

### Detailed description of the invention

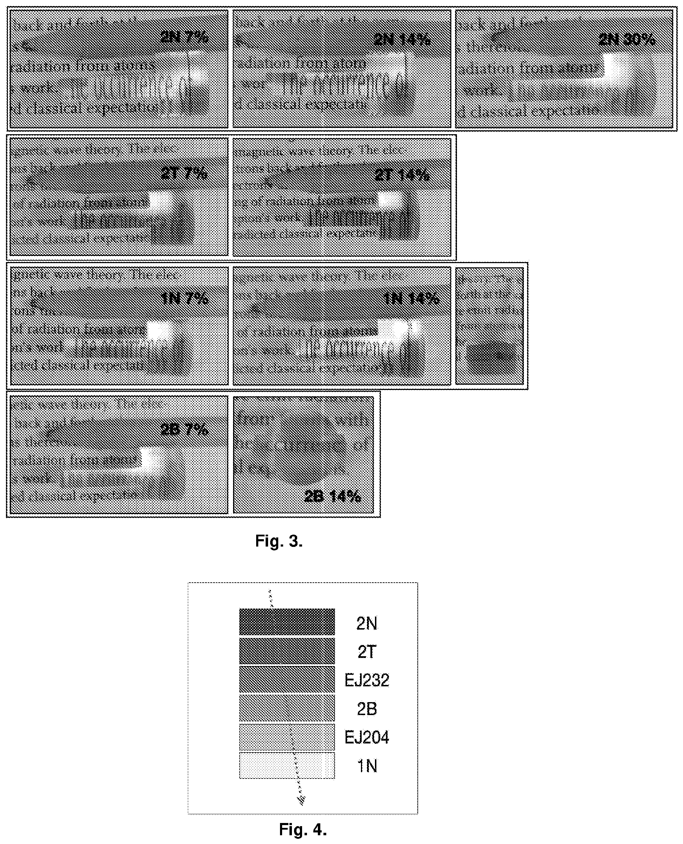

Within the scope of the present invention, the following definitions are given:
- The term **Optical photons or Fluorophores** means photons with a wavelength that falls in the domain of visible light, i.e. indicatively from 390 to 700 nm.
- The term **Primary dopants** means the chemical compounds added to the primary plastic matrix of the scintillators. They have the property of emitting optical photons isotropically when ionizing radiation passes through the compound.
- The term **Light Yield** refers to the quantity of optical photons emitted by the scintillator in relation to the energy released inside the scintillator by the ionizing radiation which passes through it. Only a small fraction of the energy loss of a charged particle in the scintillation counter is converted into visible light. This conversion factor is usually given as compared to anthracene [Sangster56, R.C. Sangster and J.W. Irwine, Study of organic scintillators, Journal. Chem. Phys. 24 (1956) 670.], whose light yield is of the order of 5% for blue light or about two photons/100 eV for high-energy particles. Typical plastic scintillators give 50-60% [Bicron93, Bicron Scintillation Products, Brochure, available from BICRON, Newbury, Ohio (USA), or P.O.B. 3093, NL-3760 DB Soest.].
- The term **Light Output** (**LO**) refers to the light response from the scintillator in terms of collected charge, compared to the energy released inside the scintillator by the ionizing radiation that passes through it.
- The term **Photo-detector** means the device that reacts (and supplies a signal) when hit by a photon. There are very different types in shape and also in the principle of operation. In our case we will consider a photo-detector which supplies an electronic signal when it is hit by light (i.e. by optical photons).
- The term **Quenching** refers to the phenomenon of saturation of the light signal emitted by the scintillator. The quenching phenomenon occurs in the event of a large release of energy from the incident radiation to the scintillating medium, which is no longer able to emit optical photons in a way proportional to the energy released by the radiation. The most direct effect of quenching is the loss of linearity between the energy released by the radiation and the number of optical photons emitted by the scintillator.
- The term **Self-absorption** means that the optical photons emitted by the scintillator can be re-absorbed by the scintillator itself before they reach the photo-detector and generate a detectable electronic signal. It can be schematized as a non-perfect transparency of the medium, which prevents the light from propagating undisturbed inside it but absorbs a certain quantity.
- The term **Secondary dopant** means the chemical compound that absorbs the optical photons emitted by the primary dopant and re-emits them with a wavelength different from the original one.
- The term **Emission band** means the wavelength range wherein optical photons are emitted, typically from ultraviolet/violet to red [300-700 nm].
- The term **Scintillation time** indicates the period of time that elapses between the passage of the radiation inside the scintillator and its emission of optical scintillation photons. In this work the characterization of the decay time has not been performed.
- The **Rise time** is generally defined as the time interval between the moment wherein the signal emitted by the device passes from 10% to 90% of its maximum value. In this work, the rise time characterization was performed by analyzing the response signal of the scintillators placed under the same conditions. In Table 1 this parameter is compared between commercial scintillators and scintillators of this invention. The measured values are all comparable and in a range of 2-3 ns.
- The term **Decay time** refers to the time interval wherein the optical photons are emitted as a consequence of the passage of the incident radiation. In this work the characterization of the decay time has not been performed.
- The term **Ionizing radiation** refers to any type of radiation which, as it passes, ionises (directly or indirectly) the atoms of the material, extracting atomic electrons from the atoms.
- The term **Pulse Shape Discrimination** or **PSD** refers to the identification technique of the ionizing radiation which exploits the different temporal response of the signal emitted by the detector according to the incident radiation. In particular, it is traditionally used to identify photons and alpha particles. It should be noted that a scintillator with good PSD capabilities does not absolutely present short scintillation time characteristics, on the contrary it shows two scintillation components, one faster (100 ns) and one slower (µs), which are used to distinguish the type of particle passing through it. They cannot therefore be used for fast time detectors.
- The term **Pulse width** means the total time width of the signal emitted by the radiation detector. It is a combination of the device's **rise time** and **decay time.** Scintillators with short pulse width are potentially more performing in terms of time response, but not necessarily, as can be seen in Table 1 where this parameter is compared between commercial scintillators and scintillators of this invention.
- The term **transparent** indicates a scintillator wherein the light produced is able to exit from the scintillator itself. In organic scintillators, the fluorescence emitted is made up of photons that have a lower energy than that absorbed and this implies a very low probability of being absorbed by the molecules of the medium. On the other hand, in case of poor solubility at high concentrations of fluorophore, non-transparency phenomena may occur. In figures 3 and 4, note the excellent transparency of the samples made for this invention at various concentrations. **Transparency,** therefore, is the physical property by which light passes through a material without appreciable dispersion. A transparent material is made up of components with a uniform refractive index and with no structural defects (voids, cracks, etc.) and a homogeneous molecular structure.

The present invention relates to an organic scintillator comprising a solid matrix wherein a fluorophore (also called primary dopant) is dispersed.

In particular, the invention relates to a transparent scintillator wherein the primary fluorophore or dopant is added in the matrix in an amount ranging from 1 to 30% by weight, preferably from 5 to 40% by weight, more preferably from 7 to 30% by weight, more preferably from 14 to 30% by weight and the secondary fluorophore (wave shifter) which can be omitted or added in an amount of about 0.01-2% by weight, preferably 0.1 to 1.0% by weight.

It's important to fulfill these quantities and the relative ratios between the primary dopant and the shifter for the performance of the scintillator both as regards the transparency characteristics and as regards the temporal resolution.

Then the primary dopant will be added:
- in quantities ≤ 40%, preferably ≤ 30% otherwise the transparency is unsatisfactory, and preferably
- in quantities of 5% or higher, for instance between 7 and 14% by weight in order to provide good temporal resolution.

For instance, the compositions based on primary dopants and wave shifters used in the known art provide scintillators which either are not transparent (for instance scintillators containing PPO in concentrations of the order of 25% or ter-phenyls) and/or provide unsatisfactory temporal resolutions.

The fluorophores or primary dopants according to the disclosure have the following general formula (M):

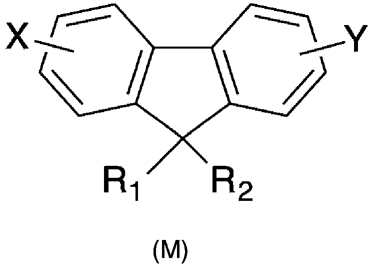

Wherein:
X and Y, independently of each other, are: H, aryl, naphthyl, biphenyl, tolyl, preferably 2-naphthyl, 1-naphthyl, 2-biphenyl, 4-tolyl; provided that X and Y are never H at the same time.
R₁ and R₂, independently of each other, are: aryl, arylalkyl, alkyl (also alkylene), with linear branched or cyclic chain C₂-C₁₂, preferably at least one of R₁ and R₂, or both, is C₈H₁₇

The known compounds of formula (M) can be prepared according to the indications given in:
Harris, J. D., Liu, J. & Carter, K. R. Synthesis of π-Bridged Dually-Dopable Conjugated Polymers from Benzylimidazole and Fluorene: Separeting Sterics from Electronics. Macromolecules, 2015, 48, 6970-6977
Yin, L., Hu, Q., Emmerich, J., Lo, M. M. C., Metzger, E., Ali, A., & Hartmann, R. W. Novel pyridyl-or isoquinolinyl-substituted indolines and indoles as potent and selective aldosterone synthase inhibitors . Journal of Medicinal Chemistry, 2014, 57(12), 5179-5189.

The invention refers to compounds with general formula (M1):

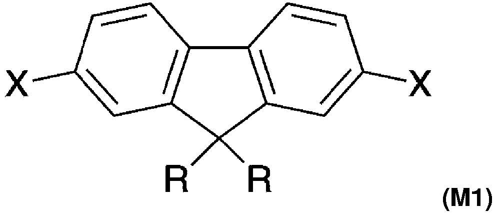

Wherein:
X = 2-naphthyl, 1-naphthyl, 2-biphenyl, 4-tolyl; R = C₈H₁₇

Particularly preferred compounds have the structural formulas selected from:

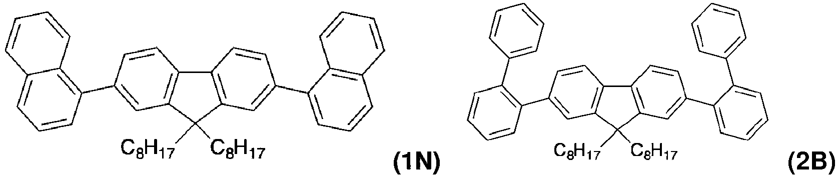

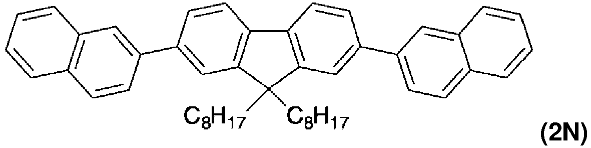

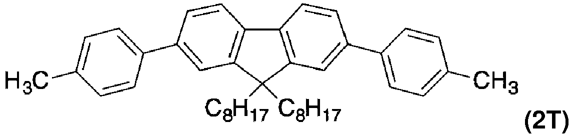

The compounds represented above can be used alone or in admixture with each other.

Scheme 1 represents the synthesis process of compounds 2T, 2B, 1N and 2N.

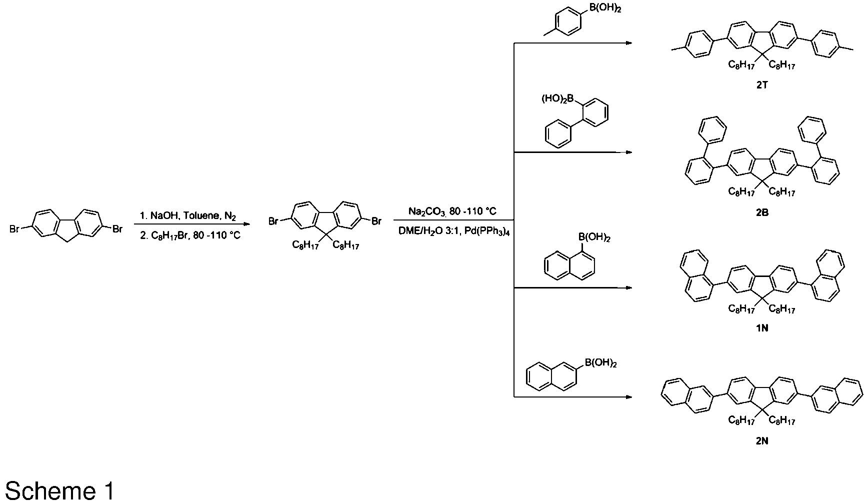

The polymeric matrices for the plastic scintillators according to the invention are known and their preparation is described in Bagán, H., et al. Crosslinked plastic scintillators: A new detection system for radioactivity measurement in organic and aggressive media. Analytica Chimica Acta, 2014, 852, 13-19. Preferred matrices of the invention are those based on polystyrene and polyvinyltoluene obtained with the crosslinkers divinylbenzene (DVB) and ethylene glycol dimethacrylate (EGDMA).

The plastic scintillators according to the invention are prepared according to methods and experimental conditions known to the person skilled in the art.

The basic stages of the procedure are as follows:
(i) Prepare a mixture comprising one or more monomers and one or more crosslinkers in quantities and operating conditions such as to obtain a final hardened and machine-workable polymeric matrix. For instance, in the case of the polyvinyltoluene (PVT) polymeric matrix, mixtures of 4-vinyltoluene can be used to which divinylbenzene (DVB, 1-5% wt) is added as crosslinker.
(ii) To the solution of step (i) are added: the primary dopant, in a concentration range from 1% to 30%, the secondary dopant or shifter (if necessary, preferably 0.1-1.0% wt) and the radical initiator, in the case of PVT 2-2'-azobisisobutyronitrile (AIBN, 0.01-0.05% wt), and solubilizes at room temperature. As secondary dopant, 7-diethylamino-4-methylcoumarin is preferred, which has excellent solubility in PVT and has therefore been used in the synthesis of the plastic scintillators of the invention.
(iii) The mixture is degassed, for instance by nitrogen flow for 20-40 minutes, placed in a closed glass vial and left in an inert atmosphere, typically at a temperature 70-90°C for 72 hours and, subsequently, 90-110°C for 24-36 hours, until complete cross-linking and final hardening.

At the end of the polymerization, the sample is left to cool slowly until it reaches room temperature; then, the glass container is destroyed and the plastic sample is treated on the surface with absolute ethyl alcohol. Subsequently the sample is subjected to polishing by means of a lapping machine using an aqueous suspension of alumina powder with a particle size of 0.05 µm (Zaitseva, N. P., et al. Recent developments in plastic scintillators with pulse shape discrimination. Nuclear Instruments and Methods in Physics Research Section A: Accelerators, Spectrometers, Detectors and Associated Equipment, 2018, 889, 97-104; Yemam, H. A., et al.Highly Soluble p-Terphenyl and Fluorene Derivatives as Efficient Dopants in Plastic Scintillators for Sensitive Nuclear Material Detection.Chemistry-A European Journal, 2017, 23(37), 8921-8931).
∘ Transmission measurements with white light show excellent transparency starting from a frequency of about 390 nm. This guarantees the high transmission efficiency of the light signal produced by the scintillator up to the photodetector and allows for the creation of centimeter-sized scintillators.
∘ Transmission luminescence measurements with a 370 nm (UV) light source show emission spectra with an average at 435 nm, for 1N*, 2B* and 2N (*doped with 7-diethylamino-4-methylcoumarin). This guarantees the possibility of using the scintillators of the invention coupled with traditional standard photo-detectors (For instance photomultipliers - PMTs) for particle detection applications in physics.
∘ From a series of experimental measurements it was possible to characterize the samples from a temporal and light production point of view.

It is necessary to underline that in a scintillator the production time of the luminous response depends on the material that is present in it at the highest concentration, because the incident radiation will primarily interact with that material. In fact, the impact on temporal resolution is not dominated by the presence or quantity of the wave shifter, but by the presence of the component with the highest concentration.

The plastic scintillators thus prepared have been subjected to various measurement campaigns, in different laboratories and with different radiation sources. The most relevant results for characterization purposes are reported in Table 1.

Plastic scintillators synthesized from new fluorenic derivatives have several advantages such as:
- extremely fast rise time and decay time
- possibility of reaching high concentrations of fluorophore while maintaining an almost complete transparency of the final plastic object
- excellent mechanical properties of the plastic object which guarantee excellent workability and ease of polishing
- highly reproducible chemical synthesis in excellent yields starting from lowcost commercial reagents
- purification procedures of the intermediate and final reaction products particularly easy and applicable on an industrial scale

The following examples are to be considered illustrative and not limiting of the scopes of the invention.

### EXAMPLES

### Total synthesis of primary dopants.

### Synthesis of 2,7-dibromo-9,9-dioctyl-9H-fluorene

In a three-necked flask, equipped with a condenser, a rubber septum and a nitrogen flow, 2.676 g (8.26 mmol) of 2,7-dibromofluorene and 0.266 g (0.826 mmol) of tetrabutylammonium bromide are dissolved in a mixture of toluene (20 mL) and NaOH(aq) (20 mL, 50% w/w). After the mixture has been degassed by nitrogen flow for approximately 15-30 minutes, 1-bromo octane (2.874 mL, 16.52 mmol) is added in 3 portions at 30-minute intervals. Once the additions are complete, the reaction is left under stirring at 80-110°C for 25-36 hours. After this time, the mixture is left to cool to room temperature and the organic phase is separated from the aqueous phase. The aqueous phase is then extracted three times with 30 mL of ethyl acetate. Once all the fractions have been combined, the organic phase thus obtained is washed with 30 mL of 1 M HCl and, subsequently, with 30 mL of a saturated aqueous solution of NaCl, then dried over Na₂SO₄, filtered, and, after having eliminated the solvent under reduced pressure, the residue is recrystallized from isopropyl alcohol to give a white solid (about 90% yield).

### Synthesis of 2,7-di(naphthyl-2-yl)-9,9-dioctyl-9H-fluorene (2N)

A suspension of 2,7-dibromo-9,9-dioctylfluorene (1 mmol), 2-naphthylboronic acid (1.5 mmol), sodium carbonate (5 mmol), and tetrakis(triphenylphosphine)palladium (0) (0.05 mmol ) in 10 mL of dimethoxyethane/water (3:1) is stirred at 80-110°C for 2-3 h under nitrogen flow. The reaction mixture is slowly cooled to room temperature and diluted with 10 mL of water. The aqueous phase is then extracted three times with 30 mL of ethyl acetate. Once all the fractions have been combined, the organic phase thus obtained is washed twice with 20 mL of a saturated aqueous solution of NaCl, then dried over Na₂SO₄, filtered, and, after having eliminated the solvent under reduced pressure, the residue is recrystallised from isopropyl alcohol to obtain a white solid in 84% yield.

### Synthesis of 2,7-di(naphthyl-1-yl)-9,9-dioctyl-9H-fluorene (1N)

A suspension of 2,7-dibromo-9,9-dioctylfluorene (1 mmol), 1-naphthylboronic acid (1.5 mmol), sodium carbonate (5 mmol), and tetrakis(triphenylphosphine)palladium (0) (0.05 mmol ) in 10 mL of dimethoxyethane/water (3:1) is stirred at 80-110°C for 2-3 h under nitrogen flow. The reaction mixture is slowly cooled to room temperature and diluted with 10 mL of water. The aqueous phase is then extracted three times with 30 mL of ethyl acetate. Once all the fractions have been combined, the organic phase thus obtained is washed twice with 20 mL of a saturated aqueous solution of NaCl, then dried over Na₂SO₄, filtered, and, after eliminating the solvent under reduced pressure, the residue is purified by silica gel chromatography using petroleum ether as eluent. A colorless oil is obtained with a yield of 88%.

### Synthesis of 2,7-di([1,1'-biphenyl]-2-yl)-9,9-dioctyl-9H-fluorene (2B)

A suspension of 2,7-dibromo-9,9-dioctylfluorene (1 mmol), 2-biphenylboronic acid (1.5 mmol), sodium carbonate (5 mmol), and tetrakis(triphenylphosphine)palladium (0) (0.05 mmol ) in 10 mL of dimethoxyethane/water (3:1) is stirred at 80-110°C for 2-3 h under nitrogen flow. The reaction mixture is slowly cooled to room temperature and diluted with 10 mL of water. The aqueous phase is then extracted three times with 30 mL of ethyl acetate. Once all the fractions have been combined, the organic phase thus obtained is washed twice with 20 mL of a saturated aqueous solution of NaCl, then dried over Na₂SO₄, filtered, and, after eliminating the solvent under reduced pressure, the residue is purified by silica gel chromatography using petroleum ether as eluent. A colorless oil is obtained with a yield of 80%.

### Synthesis of 9,9-dioctyl-2,7-di-p-tolyl-9H-fluorene (2T)

A suspension of 2,7-dibromo-9,9-dioctylfluorene (1 mmol), p-tolylboronic acid (1.5 mmol), sodium carbonate (5 mmol), and tetrakis(triphenylphosphine)palladium (0) (0.05 mmol ) in 10 mL of dimethoxyethane/water (3:1) is stirred at 80-110°C for 2-3 h under nitrogen flow. The reaction mixture is slowly cooled to room temperature and diluted with 10 mL of water. The aqueous phase is then extracted three times with 30 mL of ethyl acetate. Once all the fractions have been combined, the organic phase thus obtained is washed twice with 20 mL of a saturated aqueous solution of NaCl, then dried over Na₂SO₄, filtered, and, after having eliminated the solvent under reduced pressure, the residue is recrystallised from isopropyl alcohol to obtain a white solid in 84% yield.

### Preparation of plastic scintillators

The plastic scintillators according to the invention were prepared as follows using polyvinyltoluene (PVT) as polymeric matrix, obtained from the polymerization of the monomer 4-vinyltoluene (purchased from Sigma-Aldrich, d = 0.897 g/mL, from 3.5 mL to 6.0 mL depending on geometry type), previously purified by column chromatography using alumina (Al₂O₃) as stationary phase to remove the polymerization inhibitor 3,5-di-tertbutylcatechol (TBC).

In order to maintain excellent transparency and workability properties of the final plastic object, even at high concentrations of primary dopant, divinylbenzene was added to 4-vinyltoluene as a crosslinking agent (DVB, 1-5% wt, purchased from TCI). In this solution the primary dopant (1N, 2N, 2T or 2B, 1-30% wt), the secondary (7-diethylamino-4-methylcoumarin, purchased from J&K Scientific, 0.3%-0.5% wt), and the radical initiator 2-2'-azobisisobutyronitrile (AIBN, purchased from Sigma-Aldrich, 0.01-0.05% wt) are solubilized at room temperature.

The mixture is degassed by nitrogen flow for 20-40 minutes, placed in a closed glass vial and left in an inert atmosphere at a temperature of 79°C for 72 hours and, subsequently, at 97°C for 24-36 hours until cross-linking completed and final hardening.

At the end of the polymerization, the sample is left to cool slowly until it reaches room temperature; then, the glass container is destroyed and the plastic sample is treated on the surface with absolute ethyl alcohol. Subsequently the sample is subjected to polishing by means of a lapping machine using an aqueous suspension of alumina powder with a particle size of 0.05 µm (Zaitseva, N. P., et al. Recent developments in plastic scintillators with pulse shape discrimination. Nuclear Instruments and Methods in Physics Research Section A: Accelerators, Spectrometers, Detectors and Associated Equipment, 2018, 889, 97-104; Yemam, H. A., et al.Highly Soluble p-Terphenyl and Fluorene Derivatives as Efficient Dopants in Plastic Scintillators for Sensitive Nuclear Material Detection.Chemistry-A European Journal, 2017, 23(37), 8921-8931).

Similarly, scintillators with primary dopant 1N, 2T, 2N, 2B were prepared.

Variations of the non-limiting example described are possible, without however departing from the scope of protection of the present invention. All equivalent embodiments for a person skilled in the art are included in the scope of protection of the claims.

From the description given above, the person skilled in the art is capable of providing the object of the invention without having to introduce further details.

The experimental setup with which the scintillators prepared as indicated above (also called "samples") were used is described below.

Samples were processed with mechanical cutting (SBAI mechanical laboratory) and manual polishing with lapping machine (Buehler Metaserv Grinder Polisher).

Radiation source: cosmic rays (particles at minimum ionization). The cosmic rays that reach the earth are charged particles (muons) of very high energy (4 GeV) which have the particularity of releasing a constant amount of energy into the material they pass through which in this case depends only on the path actually traveled in the scintillators under examination. Cosmic rays, often called simply 'cosmic', are therefore energetic particles coming from the atmosphere as they are produced by the interaction of other particles coming from outer space with the matter that makes up the Earth's atmosphere. The use of cosmic rays in the characterization measurements of detectors and materials is a standard for particle physics due to their ineluctable constant and unstoppable presence. Therefore, no additional equipment is used to produce them.

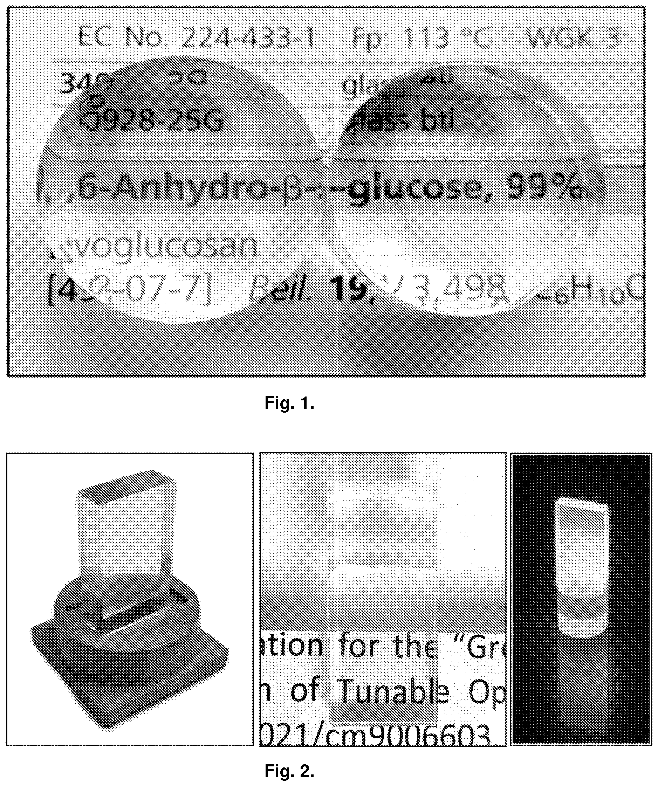

The samples were positioned horizontally so as to show the thinner side perpendicular to the direction of the cosmic rays (muons). In this way is obtained a minimization of the impact of the different pathways in the active material. Figure 5 shows a diagram of the data acquisition geometry and figure 2 shows a photo of the sample in its mechanical support.

The reading of the light produced by the scintillators is performed by fast photomultipliers (PMT Hamamatsu, H10721-20) with a rise-time of 0.57 ns.

The acquisition system is made with CAEN commercial electronics, created NIM and VME standards (www.caen.it): linear fan in/out N625, dual timer N93B, coincidence unit N, bridge V2718, waveform digitizer V1742, pci card V2818 and Dell Tower 5810 workstation.

The power supply system is Low Voltage Power Supply (Tti CPX, https://www.aimtti.com/product-category/dc-power-supplies).

The control system is a LeCroy 354 oscilloscope (https://teledynelecroy.com/oscilloscope/).

In order to estimate the performance of our samples we used two models of commercial plastic scintillators referred to as EJ-232 and EJ-204 which represent a standard in terms of optimization of the temporal and luminous performances, respectively.

The EJ-232 (Eljen Technology) plastic scintillator, with the same characteristics as the BC-422 (Saint Gobain) scintillator, is the commercial product which shows that it can achieve the best temporal resolutions.

The EJ-204 plastic scintillator (Eljen Technology), with the same characteristics as the BC-404 scintillator (Saint Gobain), is the commercial product showing the highest light output.

The most relevant results for characterization purposes are reported in Table 1.

**Table 1.**

| **Samples** | **Primary dopant** | **Emission wavelenght*** | **Light Output **% J232** | **Rise-Time [ns]** | **Width [ns]** | **Temporal Resolution [ps]** |
|---|---|---|---|---|---|---|
| | % | [nm] | *systematic and statistics error 10%* | | | |
| **EJ-232** | - | 370 | 100 | 2 | 9 | 123 |
| **EJ-204** | - | 408 | 200 | 2.5 | 11 | 211 |
| **2N** | 14% | 405 | 110 | 2 | 12 | 81 |
| **2T** | 14% | - | 240 | 3 | 18 | 97 |
| **1N** | 14% | 415 | 155 | 3 | 17 | 102 |
| **2B** | 14% | 420 | 160 | 2.5 | 14 | 110 |

| | | | | | | |
|---|---|---|---|---|---|---|
| ** Wavelength at maximum emission. For the 2T, the measurement of the emission spectrum was not carried out.* *** Luminous response obtained with the read-out system of the experimental setup used - PMT_H10721-20 - quantum efficiency peak at 400 nm.* | | | | | | |

In the table it is possible to observe that the prepared plastic scintillators show that they can reach better temporal resolutions than those relating to the commercial scintillator EJ-232, in particular the scintillator indicated with 2N reaches a 35% better temporal resolution than the commercial one.

It is worth noting that in the case of 2T, for instance, the temporal resolution obtained experimentally is in any case 20% better than the fast commercial, and 55% better than the brighter commercial, against a very significant increase in light.

This light increase allows to add to the best time measurements also an energy resolution measurement 35% better than the brightest commercial scintillator.

## Claims

1. A scintillator **characterized by** a transparent polymeric matrix and comprising one or more primary dopants selected from among fluorenic compounds having the following general formula (M1): 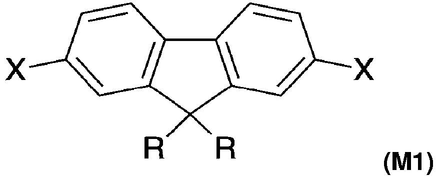 Wherein:
X = 2-naphthyl, 1-naphthyl, 2-biphenyl, 4-tolyl; R = C₈H₁₇
and wherein said one or more primary dopants are present in an amount of 5-30% by weight with respect to the polymeric matrix,
the fluorenic compounds including, in particular, the compounds having formulas (1N), (2B), (2N), and (2T).

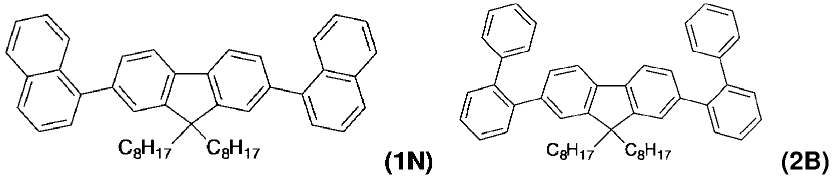

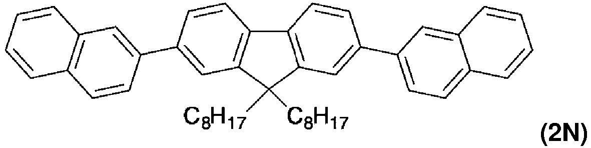

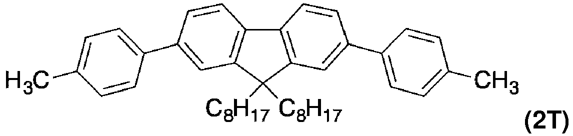

2. The scintillator according to claims 1 wherein the polymeric matrix is selected from polystyrene, cross-linked polystyrene, poly(vinyl-toluene) and polyvinylpyrrolidone.

3. The scintillator according to any one of claims 1-2 further comprising a secondary dopant, preferably 7-diethylamino-4-methylcoumarin.

4. The scintillator according to any one of claims 1-3 **characterized by** a rise time lower than 350 ps, a pulse width lower than 20 ns, against a Light yield (LO) of about 55% or higher as compared to anthracene, the parameters being measured as described in the description.

5. The scintillator according to claim 4 **characterized by** a time resolution lower than 120 ps, preferably lower than 100 ps determined as described in the description.

6. A fluorenic compound selected from the compounds having the formulas:

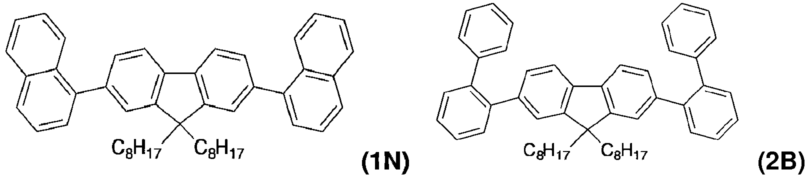

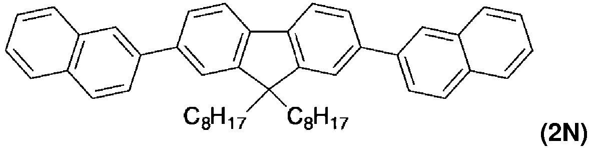

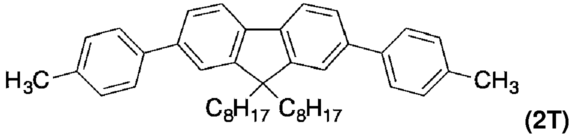

7. A particle detection device which uses the scintillator of the preceding claims.

8. The detection device according to claim 7 wherein the particles are selected from: electrons, photons, protons, leptons and hadrons.

9. Use of the fluorenic compound of claim 6 as primary dopants in plastic organic scintillators.

## Patentansprüche

1. Szintillator, **gekennzeichnet durch** eine transparente Polymermatrix und einen oder mehrere primäre Dotierstoffe, ausgewählt aus folgenden Fluorenverbindungen mit der allgemeinen Formel (M1): 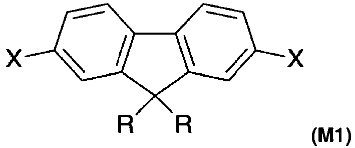 Dabei gilt:
X = 2-Naphthyl, 1-Naphthyl, 2-Biphenyl, 4-Tolyl; R = C₈H₁₇ und
wobei einer oder mehrere der genannten primären Dotierstoffe in einer Menge von 5 bis 30 Gew.-% bezogen auf die Polymermatrix vorliegen,
wobei die Fluorverbindungen insbesondere die Verbindungen mit den Formeln (1N), (2B), (2N), und (2T) umfassen,

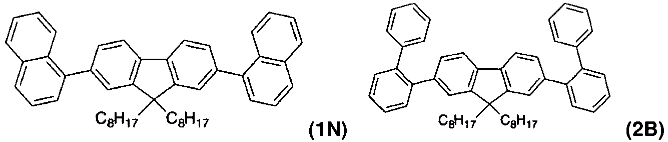

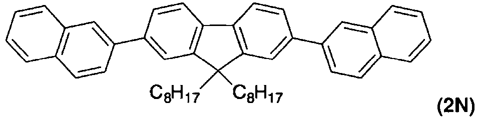

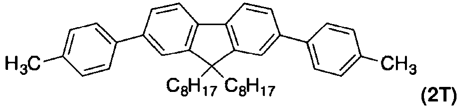

2. Szintillator nach Anspruch 1, wobei die Polymermatrix ausgewählt ist aus Polystyrol, vernetztem Polystyrol, Poly(vinyl-toluol) und Polyvinylpyrrolidon.

3. Szintillator nach einem der Ansprüche 1 oder 2, ferner umfassend ein sekundäres Dotierungsmittel, vorzugsweise 7-Diethylamino-4-methylcumarin.

4. Szintillator nach einem der Ansprüche 1 bis 3, **gekennzeichnet durch** eine Anstiegszeit von weniger als 350 ps, eine Impulsbreite von weniger als 20 ns, und eine Lichtausbeute (LO) von ca. 55 % oder mehr relativ zu Anthracen, wobei die Parameter wie in der Beschreibung beschrieben gemessen werden.

5. Szintillator nach Anspruch 4, **gekennzeichnet durch** eine zeitliche Auflösung von weniger als 120 ps, vorzugsweise weniger als 100 ps, bestimmt wie in der Beschreibung beschrieben.

6. Fluorenverbindung, ausgewählt aus Verbindungen der folgenden Formeln:

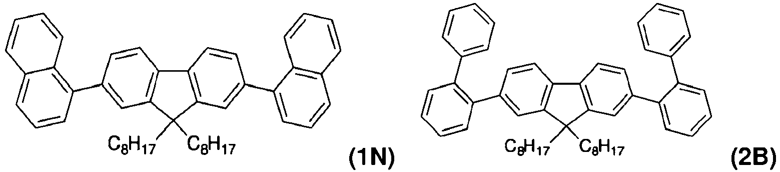

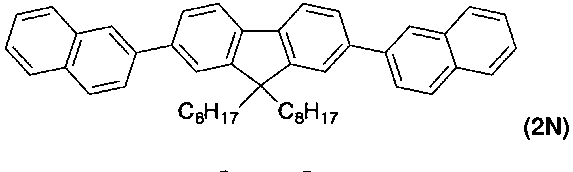

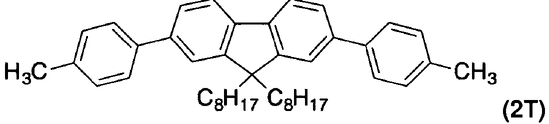

7. Partikeldetektorvorrichtung, die den Szintillator nach den vorhergehenden Ansprüchen verwendet.

8. Detektorvorrichtung nach Anspruch 7, wobei die Partikel ausgewählt sind aus: Elektronen, Photonen, Protonen, Leptonen und Hadronen.

9. Verwendung der Fluorenverbindungen nach Anspruch 6 als primäre Dotierstoffe in organischen Kunststoffszintillatoren.

## Revendications

1. Scintillateur **caractérisé par** une matrice polymère transparente et comprenant un ou plusieurs dopants primaires, choisis parmi des composés fluoréniques de la formule générique suivante (M1): 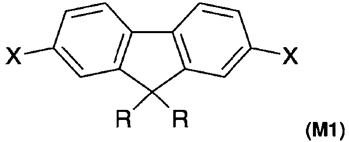 dans lequel:
X = 2-naphtyle, 1-naphtyle, 2-biphényle, 4-tolyle; R = C₈H₁₇
et dans lequel l'un ou plusieurs lesdits dopants primaires sont présents en une quantité de 5 à 30% en poids par rapport à la matrice polymérique,
les composés fluoréniques comprenant en particulier les composés avec les formules (1N), (2B), (2N), e (2T).

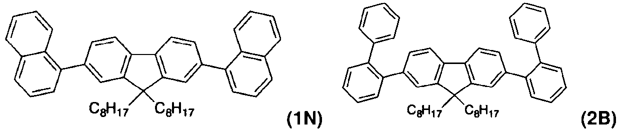

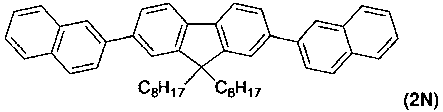

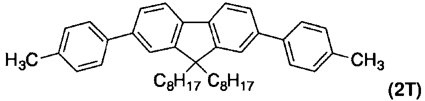

2. Scintillateur selon la revendication 1, dans lequel la matrice polymère est choisie parmi polystyrène, polystyrène reticulé, poly(vinyltoluène) et polyvinylpyrrolidone.

3. Scintillateur selon l'une quelconque des revendications 1 à 2, comprenant en outre un dopant secondaire, de préférence 7-diéthylamino-4-méthylcoumarine.

4. Scintillateur selon l'une quelconque des revendications 1 à 3, **caractérisé par** un temps de montée inférieur à 350 ps, une largeur d'impulsion inférieure à 20 ns, par rapport à un rendement lumineux (RL) d'environ 55% ou plus par rapport à l'anthracène, les paramètres étant mesurés comme décrit dans la description.

5. Scintillateur selon la revendication 4, **caractérisé par** une résolution temporelle inférieure à 120 ps, de préférence inférieure à 100 ps, déterminée comme décrit dans la description.

6. Composé fluorénique choisi parmi les composés de formules suivantes:

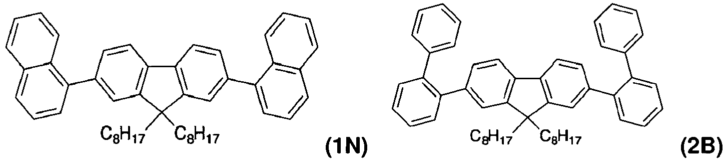

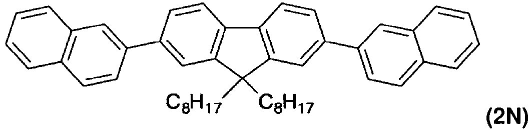

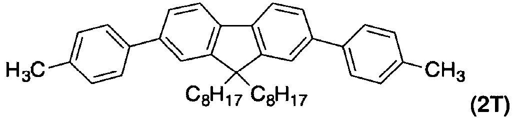

7. Dispositif de détection de particules utilisant le scintillateur des revendications précédentes.

8. Dispositif de détection selon la revendication 7, dans lequel les particules sont choisies parmi: électrons, photons, protons, leptons et hadrons.

9. Utilisation des composé fluorénique selon la revendication 6, comme des dopants primaires dans les scintillateurs organiques plastiques.
